(19) Europäisches Patentamt
European Patent Office
Office européen des brevets

(11) Veröffentlichungsnummer: **0 103 776**
**A2**

(12) # EUROPÄISCHE PATENTANMELDUNG

(21) Anmeldenummer: **83108364.7**

(22) Anmeldetag: **25.08.83**

(51) Int. Cl.³: **C 07 D 235/32**
C 07 D 253/06, C 07 D 277/82
C 07 C 127/24, C 07 D 249/08
A 01 N 43/52, A 01 N 43/64
A 01 N 43/78

(30) Priorität: **04.09.82 DE 3232917**
**15.10.82 DE 3238358**

(43) Veröffentlichungstag der Anmeldung:
**28.03.84 Patentblatt 84/13**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Richter, Roland, Dr.**
**Roggendorfstrasse 53**
**D-5000 Köln 80(DE)**

(72) Erfinder: **Reinecke, Paul, Dr.**
**Lessingstrasse 11**
**D-5090 Leverkusen 3(DE)**

(72) Erfinder: **Müller, Hanns Peter, Dr.**
**Im Kerberich 6**
**D-5068 Odenthal(DE)**

(72) Erfinder: **Bonse, Gerhard, Dr.**
**Wolfskaul 3**
**D-5000 Köln 80(DE)**

(72) Erfinder: **Brandes, Wilhelm, Dr.**
**Eichendorffstrasse 3**
**D-5653 Leichlingen 1(DE)**

(54) **Neue Verbindungen, Verfahren zu ihrer Herstellung sowie ihre Verwendung als Schädlingsbekämpfungsmittel mit verlängerter Wirkdauer.**

(57) Die Erfindung betrifft Verfahren zur Herstellung neuer Wirkstoffe aus Wirkstoffen mit mindestens einem aminischen, hydrazinischen oder guanidinischen NH-Rest oder mindestens einem alkoholischen oder phenolischen OH- oder SH-Rest oder mindestens einem primären oder sekundären Amidrest oder einem Harnstoffrest mit mindestens einer freien NH-Gruppe, dadurch gekennzeichnet, daß sie hergestellt werden, indem man einen Wirkstoff über eine der zuvor erwähnten Gruppen mit einem erfindungsgemäßen Polyetherisocyanat verknüpft.

EP 0 103 776 A2

BAYER AKTIENGESELLSCHAFT 5090 Leverkusen, Bayerwerk

Zentralbereich
Patente, Marken und Lizenzen Rt/m-c
(Ia)

# Neue Verbindungen, Verfahren zu ihrer Herstellung sowie ihre Verwendung als Schädlingsbekämpfungsmittel mit verlängerter Wirkdauer

Die vorliegende Erfindung betrifft neue Verbindungen, ein Verfahren zu ihrer Herstellung sowie ihre Verwendung als Schädlingsbekämpfungsmittel mit verlängerter Wirkdauer.

Mittel mit verlängerter Wirkdauer, d.h. mit verzögerter Wirkstofffreisetzung (Slow-Release-Verhalten), sind Verbindungen, in denen ein Wirkstoffmolekül chemisch an einen polymeren Träger gebunden ist und die unter Anwendungsbedingungen die aktive Wirkstoffkomponente durch Hydrolyse oder Depolymerisation aus dem polymeren Träger freisetzen.

Bekannte Verfahren zur Herstellung solcher Verbindung sind zum Beispiel die Verknüpfung eines, eine reaktive Gruppe enthaltenden, Wirkstoffs (z.B. Isocyanat-Gruppe) mit einem geeigneten polymeren Träger wie Polyvinylalkohol (US-PS. 4 267 281) oder mit einem polymerisierbaren Monomer wie Acrylsäure (US-PS 4 225 693) oder mit einem Glycidylgruppen aufweisenden Copolymerisat (DE-OS 2 819 340). Der Nachteil dieser Verfahren besteht darin, daß entweder der Wirkstoff zur Funktionalisierung chemisch umgewandelt werden muß, z.B. durch Überführung einer Amingruppe in eine Isocyanatgruppe, was mit Verlust an teurer Wirksubstanz verbunden ist oder ausschließlich nur Wirkstoffe, die eine Hydroxylgruppe enthalten, derivatisiert werden können oder bei nachfolgender Polymerisation neben der Hydrolyse zusätzliche Faktoren wie Depolymerisationsgeschwindigkeit und Diffusion aus dem Polymergerüst die Freisetzung des im Polymer eingebetteten Wirkmoleküls beeinflussen, was die Anwendung wegen der schlechten Reproduzierbarkeit erheblich einschränkt.

Es ist bekannt, Wirkstoffe, die ein Zerewitinoff-aktives Wasserstoffatom besitzen, über ein Koppelglied, welches zwei, gegenüber Zerewitinoff-aktiven Wasserstoffatomen, reaktive Gruppen besitzt, mit einem OH- oder NH-monofunktionellen Polyether zu verknüpfen; vgl. DE-OS 2 901 060, DE-OS 2 910 356 und DE-OS 2 912 289. Dieses Verfahren soll dazu dienen, die Löslichkeit von biologisch aktiven Wirkstoffen in Wasser und niederen aliphatischen Alkoholen zu verbessern. Die Wirkstoffe behalten trotz einer das Molekulargewicht veränderten Modifizierung ihre volle Wirkung, ja, sie erlangen sogar eine zusätzliche systemische Wirkung.

Über eine Verlängerung der Wirkdauer von Pflanzenschutzmitteln durch diese Derivatisierung ist nichts bekannt.

Gegenstand der vorliegenden Erfindung sind neue Verbindungen, dadurch gekennzeichnet, daß sie herstellbar sind, indem man Schädlingsbekämpfungsmittel mit mindestens einem Zerewitinoff-aktiven Wasserstoffatom mit Polyetherisocyanaten umsetzt, unter der Voraussetzung, daß die Umsetzung mit dem Polyetherisocyanat nicht an einem Wasserstoffatom erfolgt, das Teil einer Carbaminsäureestergruppe $(-O-\overset{O}{\overset{\|}{C}}-NH-)$ ist.

Gegenstand der vorliegenden Erfindung ist außerdem das Verfahren zur Herstellung der neuen Verbindungen, das dadurch gekennzeichnet ist, daß man Schädlingsbekämpfungsmittel mit mindestens einem Zerewitinoff-aktiven Wasserstoffatom mit Polyetherisocyanaten umsetzt, unter der Voraussetzung, daß die Umsetzung mit dem Polyetherisocyanat nicht an einem Wasserstoffatom erfolgt, das Teil einer Carbaminsäureestergruppe $(-O-\overset{O}{\overset{\|}{C}}-NH-)$ ist.

Unter dem Begriff Zerewitinoff-aktives Wasserstoff versteht man ein Wasserstoffatom, welches in einer organischen Verbindung an ein Zentrum gebunden ist, welches, verglichen mit einem C-Atom eines Kohlenwasserstoffs, eine stark elektronenabziehende Wirkung ausübt. Im engeren Sinne bedeutet Zerewitinoff-aktiv ein im Sinne der Reaktion: $CH_3MgJ + H-X \longrightarrow CH_4 + JMgX$ aktives H-Atom (s.a. Beyer "Lehrbuch der organischen Chemie" (1968) Seite 147).

Unter dem Begriff Schädlingsbekämpfungsmittel versteht man Insektizide, Akarizide, Nematizide, Fungizide, Bakterizide, Mikrobizide, Virizide, Algizide, Herbizide, Pflanzenwachstumsregulatoren sowie das Wachstum einzelner oder aller Stadien in der Entwicklung von Insekten oder Akariden beeinflussender Verbindungen. Diese Mittel finden auf den Gebieten der Land- und Forstwirtschaft sowie auf dem Haushalts- und Hygiene- und Tierzuchtsektor Verwendung.

Die erfindungsgemäßen Verbindungen besitzen eine gegenüber den Ausgangswirkstoffen verlängerte Wirkdauer. Bei Einsatz der neuen Wirkstoffe erübrigt sich damit in vielen Fällen eine mehrfache Aufbringung ein und desselben Wirkstoffs. Die Verwendung der neuen Verbindungen richtet sich nach der Verwendung der als Ausgangsstoff zu ihrer Herstellung eingesetzten Schädlingsbekämpfungsmittel.

Bevorzugt seien folgende Schädlingsbekämpfungsmittel, die als Ausgangsstoffe dienen, genannt:

a) Wirkstoffe, die eine oder mehrere aminische Gruppen enthalten, die mindestens einen freien NH-Rest aufweisen, wobei der NH-Rest Teil eines Heterocyclus sein kann,

b) Wirkstoffe, die eine oder mehrere Hydrazingruppen enthalten, die mindestens einen freien NH-Rest aufweisen, wobei die Hydrazingruppe Teil eines Heterocyclus sein kann,

c) Wirkstoffe, die eine oder mehrere Guanidingruppen enthalten, die mindestens einen freien NH-Rest aufweisen, wobei die Guanidingruppe Teil eines Heterocyclus sein kann,

d) Wirkstoffe, die eine oder mehrere alkoholische Hydroxyl- oder Mercapto-Gruppen enthalten,

e) Wirkstoffe, die eine oder mehrere phenolische Hydroxyl- oder Mercapto-Gruppen enthalten,

f) Wirkstoffe, die eine oder mehrere Carbonsäureamid-Gruppen enthalten, die mindestens einen freien NH-Rest aufweisen, wobei die Carbonsäureamidgruppe Teil eines Heterocyclus sein kann,

g) Wirkstoffe, die einen oder mehrere Harnstoff-Gruppen enthalten, die mindestens einen freien NH-Rest aufweisen, wobei die Harnstoffgruppe Teil eines Heterocyclus sein kann.

Im einzelnen seien genannt:

Aus Gruppe a)
Wirkstoffe, die eine freie primäre oder sekundäre Aminogruppe beinhalten, wie z.B. N-(N'-6-Aminophenyl-thiocarbamoyl)-carbaminsäuremethylester, Heterocyclen mit freien Aminogruppen wie 2-Amino-1,3,4-thiadiazole, 5-Amino-5-chlor- bzw. brom-2-phenyl-pyridazin-3-one oder 4-Chlor-5-methylamino-2-(4-trifluormethylphenyl)-pyridazin-3-on;

Aus Gruppe b):

Wirkstoffe aus der Reihe der 4-Amino-1,2,4-triazine wie z.B. 4-Amino-6-tert.-butyl-3-(methylthio)-1,2,4-triazin-5(4H)-on und 4-Amino-3-methyl-6-phenyl-1,2,4-triazin-5(4H)-on oder Maleinsäurehydrazid und 3-Methyl-4-(2-chlorphenylhydrazone)-1,2-oxazolon-(5), 0,0-Diethyl-0-(3-methyl-5-pyrazolyl)-thionophosphorsäureester.

Aus Gruppe c):

Wirkstoffe wie z.B. 3-Amino-1,2,4-triazol, 2-Chlor-4-ethylamino-6-isopropylamino-1,3,5-triazin, 2-Chlor-4-ethylamino-6-(1-cyano-isopropylamino)-1,3,5-triazin, 6-(2-Chloranilino)-2,4-dichlor-1,3,5-triazin und 2-Benzimidazolcarbaminsäuremethylester sowie 4- bzw. 5-Methylbenzimidazolcarbaminsäuremethylester.

Aus Gruppe d):

Wirkstoffe aus der Reihe der Diphenylcarbinole wie z.B. 1,1-Bis-(4-chlorphenyl)-2,2,2-trichlorethanol und 2,2-Bis-(4-chlorphenyl)-2-hydroxy-essigsäure-isopropylester, Hydroxy-Gruppen enthaltende Phosphonsäureester wie z.B. 0,0-Dimethyl-(1-hydroxy-2,2,2-trichlorethyl)-phosphonsäureester oder 3,3-Dimethyl-2-hydroxy-1-(4'-phenyl-phenoxy)-1-(1,2,4-triazol-1-yl)-butan oder 9-(Carbomethoxy)-2-chlor-9-hydroxy-fluoren und 4-Hydroxy-3-(1,2,3,4-tetrahydro-naphth-1-yl)-2H-chromenon.

Aus Gruppe e):

Wirkstoffe wie z.B. 6-tert.-Butyl-2,4-dinitrophenol oder Heteroaromaten, die Hydroxy-Gruppen tragen wie

z.B. 3-Hydroxy-5-methyl-1,2,-oxazol, 5-Butyl-2-(dimethylamino)-4-hydroxy-6-methyl-pyrimidin oder 5-Butyl-2-(ethylamino)-4-hydroxy-6-methyl-pyrimidin.

Aus Gruppe f):
Wirkstoffe aus der Reihe der Phosphorsäureester wie z.B. O,O-Dimethyl-S-(methylaminocarbonyl-methylen)-dithiophosphorsäureester und O,O-Dimethyl-S-(methylaminocarbonyl-methylen)-thiophosphorsäureester oder das Phosphorsäureamid O,S-Dimethyl-thionophosphorsäureamid, sowie Heterocyclen, bei denen die Amid-Struktur ein Teil des Heterocyclus ist wie 2-Thiono-4-oxo-1,3-thiazolidin.

Aus Gruppe g):
Wirkstoffe aus der Reihe der 3-Aryl-1,1-dimethylharnstoffe wie z.B. 3-(3,4-Dichlorphenyl)-1,1-dimethylharnstoff oder Harnstoffe, die einen Heterocyclus als Substituent enthalten wie z.B. 1-Isobutylaminocarbonyl-2-imidazolidinon, 1,3-Dimethyl-1-(5-tert.-butyl-1,3,4-thiadiazol-2-yl)-harnstoff, 1,3-Dimethyl-1-(5-trifluormethyl-1,3,4-thiadiazol-2-yl)-harnstoff und 1-(Benzo-1,3-thiazol-2-yl)-1,3-dimethylharnstoff; weiterhin Wirkstoffe, deren Harnstoff-Struktur vollständig als Teil des Heterocyclus vorliegt, wie z.B. 4-Trichlormethylmercapto-3,5-dioxo-1,2,4-triazolidin, 3-(2-Butyl)-5-brom-6-methyl-uracil oder 3-Cyclohexyl-5,6-trimethylenuracil sowie Acylharnstoffe der allgemeinen Formel (I),

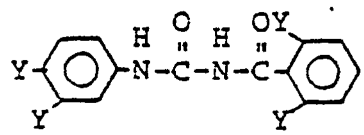

(I)

worin

Y für Wasserstoff, Halogen und Halogenalkyl oder Halogenalkoxy mit 1 - 4 C-Atomen steht.

Als Beispiele seien besonders genannt:
Herbizide, wie z.B. 4-Amino-6-tert.-butyl-3-(methylthio)-1,2,4-triazin-5(4H)-on, 4-Amino-3-methyl-6-phenyl-1,2,4-triazin-5(4H)-on, 3-(3,4-Dichlorphenyl)-1,1,-dimethylharnstoff, 1-(Benzo-1,3-thiazol-2-yl)-1,3-dimethylharnstoff und 1,3-Dimethyl-1-(5-ethylsulfonyl-1,3,4-thiadiazol-2-yl)-harnstoff;
Insektizide, wie z.B. 0,0-Dimethyl-(1-hydroxy-2,2,2-tirchlorethyl)-phosphonsäureester und 0,S-Dimethyl-thionophosphorsäureamid und
Fungizide, wie z.B. 2-(2-Furyl)-benzimidazol, 6-(2-Chloranilino)-2,4-dichlor-1,3,5-triazin, 3,3-Dimethyl-2-hydroxy-1-(4'-phenyl-phenoxy)-1-(1,2,4-triazol-1-yl)-butan und 2-Benzimidazolcarbaminsäuremethylester.

Ganz besonders zu erwähnen sind die fungiziden Wirkstoffe aus der Reihe der 2-Benzimidazol-carbaminsäurealkylester der allgemeinen Formel (II),

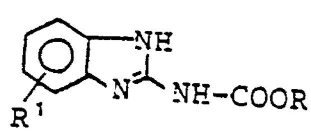

(II)

worin

R für Alkyl mit 1-4 C-Atomen und
$R^1$ für Alkyl mit 1-6 C-Atomen oder Wasserstoff steht.

Bevorzugt seien folgende als Ausgangsstoffe dienende Polyetherisocyanate der allgemeinen Formel (III) genannt

$$OCN-\left[CH_3-\overset{A}{\overset{|}{C}}H-O\right]_n-B \qquad (III)$$

in welcher

A für Wasserstoff oder Methyl,
n für ganze Zahlen zwischen 1 und 101 und
B für $C_{1-4}$-Alkyl oder für den Rest $-C_{1-4}$-Alkyl-NCO steht.

Die Herstellung der Polyetherisocyanate ist bekannt. Man erhält sie z.B. durch Phosgenierung von entsprechenden Polyethern mit Amino-Endgruppen wie in US-PS 3 370 077 und in US-PS 4 313 764 beschrieben oder durch Umwandlung der Aminopolyether in Harnstoffe und Urethane, die dann thermisch zu den entsprechenden Isocyanaten gespalten werden (DE-OS 2 943 481, 2 943 551, 3 047 898).

Bevorzugte Verwendung finden monofunktionelle Polyetherisocyanate der allgemeinen Formel (III), in denen

n = 2 - 7, A = Wasserstoff und B = $C_{1-4}$-Alkyl ist. Diese Polyetherisocyanate sind flüssige, einheitliche Verbindungen, die bei einem genau definierten Siedepunkt destillierbar sind.

Im einzelnen seien bevorzugt genannt:
3,6-Dioxaheptylisocyanat, 3,6-Dioxadecylisocyanat und 3,6,9-Trioxadecylisocyanat.

Die erfindungsgemäßen Verbindungen können vorteilhaft in der nachstehend beschriebenen Weise hergestellt werden:

Verwendet man beispielsweise 3,6-Dioxadecylisocyanat und 2-Benzimidazolcarbaminsäuremethylester (BCM) als Wirkstoff, so ist das erfindungsgemäße Verfahren wie folgt durchzuführen:

In einer Rührapparatur löst bzw. suspendiert man den Wirkstoff in einem inerten, wasserfreien Lösungsmittel, z.B. Toluol, und versetzt mit dem Isocyanat in äquimolarer Menge. Unter Feuchtigkeitsausschluß rührt man dann bei 25 bis 140°C, bevorzugt 60 bis 110°C. Die Umsetzung ist vollständig abgelaufen, wenn in der Reaktionsmischung IR-spektroskopisch kein Isocyanat mehr nachgewiesen werden kann bzw. wenn der vorher suspendierte Ausgangsstoff völlig gelöst ist. Die Aufarbeitung erfolgt in üblicher Weise, z.B. durch Abdestillieren des Lösungsmittels. Die neue Verbindung besitzt die Formel (IV)

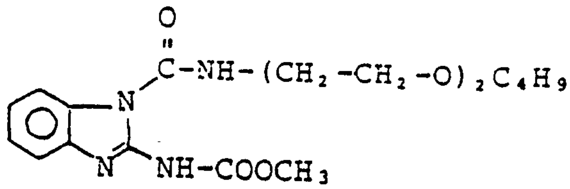

(IV)

In analoger Weise können die anderen erfindungsgemäßen Verbindungen hergestellt werden, wobei bei den als Ausgangsstoffen dienenden Wirkstoffen mit wenig basischem NH wie Harnstoffen oder Amiden der Zusatz von 0,1 bis 1 Mol-% eines die Umsetzung beschleunigenden Katalysators, wie sie aus der Polyurethan-Chemie bekannt sind, z.B. Dibutylzinndilaurat oder Zinnoctoat, angebracht sein kann.

Als Lösungsmittel werden bei der Herstellung der erfindungsgemäßen Verbindungen verwendet: Kohlenwasserstoffe wie Toluol, Xylol, Chlorkohlenwasserstoffe wie $CCl_4$, $CHCl_3$ und $CH_2Cl_2$, Ester und Ketone wie Essigsäureethylester, Butylacetat, Aceton, Ethylmethylketon, Ether wie Dioxan, Tetrahydrofuran oder Di-isopropylether sowie Acetonitril und DMF.

Als Katalysatoren dienen neben den bereits genannten Dibutylzinndilaurat, Zinndioctoat, Zinkacetylacetonat sowie tert. Amin wie Triethylendiamin, 1,8-Diazabicyclo-/5,4,0/-undec-7-en oder Dimethylcyclohexylamin.

Die Reaktion erfolgt im allgemeinen bei Normaldruck. Die Ausgangsstoffe werden im allgemeinen in äquimolaren Verhältnissen eingesetzt. Ein Überschuß der einen oder anderen Komponente bringt keine Vorteile.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Herstellungsbeispiele

Im folgenden wird das erfindungsgemäße Verfahren anhand einiger Beispiele erläutert, ohne jedoch den Erfindungsgegenstand auf diese Beispiele zu beschränken.

Beispiel 1

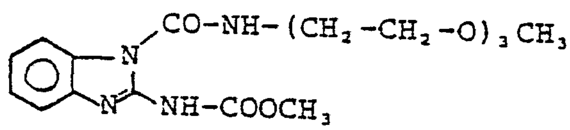

95,6 g (0,5 Mol) 2-Benzimidoylcarbaminsäuremethylester werden in 1000 ml wasserfreiem Toluol suspendiert, mit 94,5 g (0,5 Mol) 3,6,9-Trioxadecylisocyanat versetzt und unter Intergas und Feuchtigkeitsausschluß bei 80°C 7 Stunden gerührt. Nach dieser Zeit ist im IR-Spektrum die Isocyanat-Bande bei 2260 $cm^{-1}$ verschwunden und das Produkt ist bis auf einen minimalen Rest in Lösung gegangen. Dann filtriert man von unumgesetzten Wirkstoff ab und destilliert im Vakuum anschließend das Toluol vollständig ab. Man erhält 180,3 g (=95 % d.Th.) eines Feststoffs mit einem Schmelzpunkt von 148 - 150°C. Das Addukt zeigt im Massenspektrum ein stabiles $MH^{\oplus}$-Molekülion 381; Wirkstoffgehalt: 50 %.

Nach der in Beispiel 1 angegebenen Methoden werden auch die in den folgenden Beispielen aufgeführten Stoffe hergestellt.

Beispiel 2

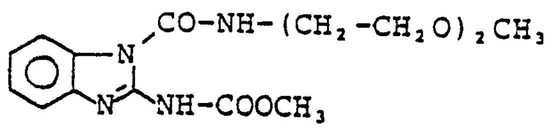

Aus 95,6 g (0,5 Mol) 2-Benzimidazolcarbaminsäuremethylester und 72,5 g (0,5 Mol) 3,6-Dioxaheptylisocyanat erhält man 157 g (=95 % d. Th.) 1-(3,6-Dioxaheptylcarbamoyl)-2-benzimidazolcarbaminsäuremethylester der Molmasse 336 ($MH^{\oplus}$ im MS).

Beispiel 3

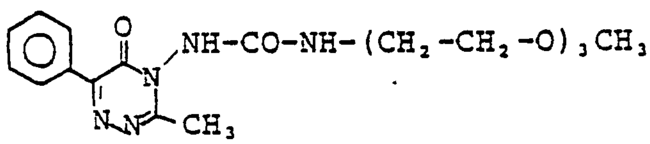

Aus 101 g (0,5 Mol) 4-Amino-3-methyl-6-phenyl-1,2,4-triazin-5(4H)-on und 94,5 g (0,5 Mol) 3,6,9-Trioxydecylisocyanat erhält man 189 g (=97 % d. Th.) eines viskosen Produkts der Molmasse 391 ($MH^{\oplus}$ im MS).

Beispiel 4

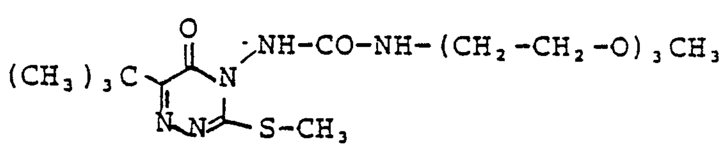

Aus 107 g (0,5 Mol) 4-Amino-6-tert.-butyl-3-(methylthio)-1,2,4-triazin-5(4H)-on und 94,5 g (0,5 Mol) 3,6,9-Trioxadecylisocyanat erhält man 197 g (= 98 % d. Th.)

eines viskosen Produkts der Molmasse 403 ($MH^{\oplus}$ im MS).

Beispiel 5

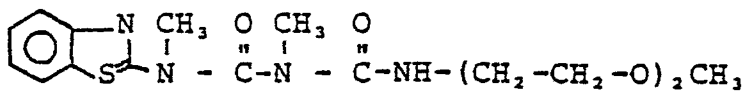

Aus 110,5 g (0,5 Mol) 1-(Benzo-1,3-thiazol-2-yl)-1,3-dimethylharnstoff und 72,5 g (0,5 Mol) 3,6-Dioxaheptylisocyanat erhält man 109 g (= 60 % d. Th.) eines Feststoffs der Molmasse 366 ($M^{\oplus}$ im MS).

Beispiel 6

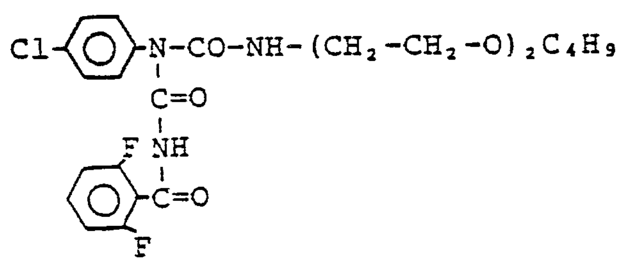

Aus 155 g (0,5 Mol) N'-(4-Chlorphenyl)-N-(2,6-difluorbenzoyl)-harnstoff und 93,5 g (0,5 Mol) 3,6-Dioxydecylisocyanat erhält man 216 g (=87 % d. Th.) eines Feststoffs, aus dessen $^{1}$H-NMR-Daten hervorgeht, daß der Acylharnstoff das Isocyanat am arylsubstituierten NH addiert und ein N-Acyl-biuret bildet. Die Molmasse beträgt 497 ($M^{\oplus}$ im MS).

Beispiel 7

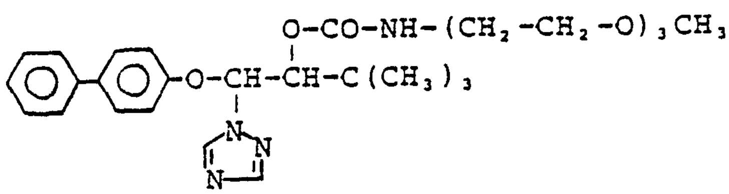

Aus 168,5 g (0,5 Mol) 3,3-Dimethyl-2-hydroxyl-1-(4-phenyl-phenoxy)-1-(1,2,4-triazol-1-yl)-butan und 94,5 g (0,5 Mol) 3,6,9-Trioxadecylisocyanat erhält man 260 g (=99 % d. Th.) eines Feststoffs der Molmasse 526 (Im Massenspektrum findet man (M-Isobuten)$^{\oplus}$ 470).

Beispiel 8

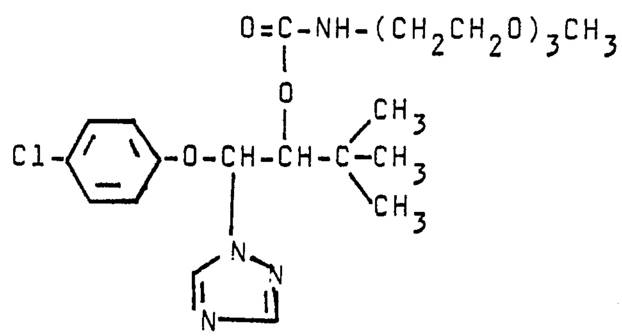

Aus 14,8 g (0,05 Mol) 1-(4-Chlorphenoxy)-3,3-dimethyl-1-(1,2,4-triazol-1-yl)-butan und 9,5 g (0,05 Mol) 3,6,9-Trioxadecylisocyanat erhält man 20,8 g (= 87 % d. Th.) 1-(4-Chlorphenoxy)-3,3-dimethyl-1-(1,2,4-triazol-1-yl)-2-(3,6,9-trioxadecylcarbonyl)-butan als viskoses Produkt der Molmasse 485 ($M^+$ im MS).

Beispiel 9

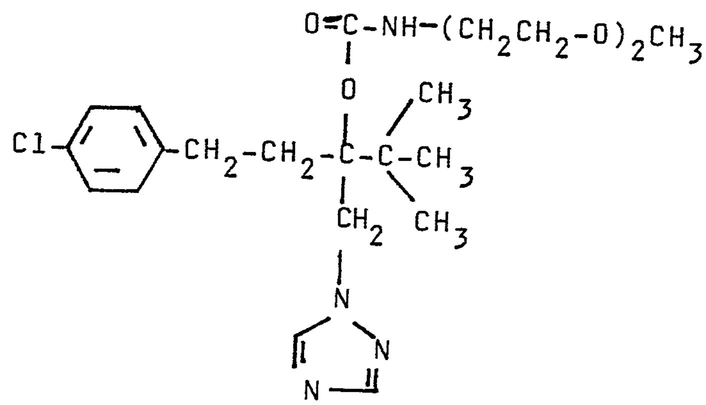

Aus 15,4 g (0,05 Mol) 1-(4-Chlorphenyl)-4,4-dimethyl-3-(methylen-1,2,4-triazol-1-yl)-petntan und 7,25 g (0,05 Mol) 3,6-Dioxaheptylisocyanat erhält man 21,7 g (96 % d. Th.) 1-(4-Chlorphenyl)-44,4-dimethyl-3-(3,6-dioxaheptylcarbonyl)-3-(methylen-1,2,4-triazol-1-yl)-pentan als viskoses Produkt der Molmasse 453 ($M^+$ im MS).

Beispiel 10

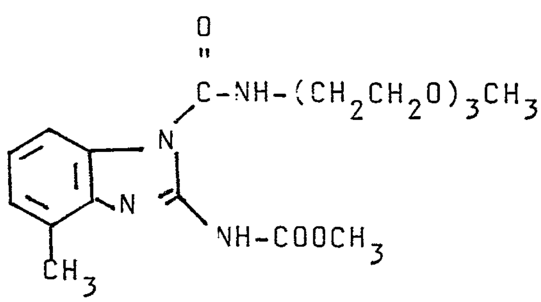

Aus 51,2 g (0,25 Mol) 4-Methyl-2-benzimidazolylmethylcarbamat und 47,3 g (0,25 Mol) 3,6,9-Trioxadecylisocyanat erhält man 94,5 g (= 96 % d. Th.) 4-Methyl-1-(3,6,9-trioxadecylcarbamoyl)-2-benzimidazolylmethylcarbamat der Molmasse 394 ($M^+$ - 32 im MS) als Feststoff mit einem Schmelzpunkt von 134°C.

Beispiel 11

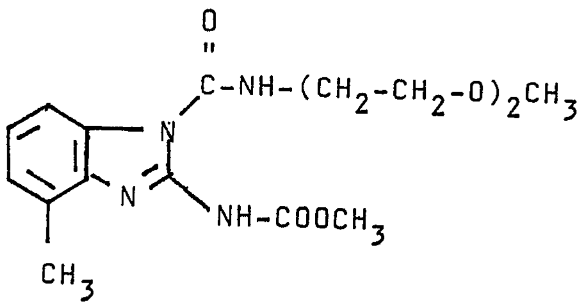

Aus 51,2 g (0,25 Mol) 4-Methyl-2-benzimidazolylmethylcarbamat und 36,3 g (0,25 Mol) 3,6-dioxaheptylisocyanat erhält man 84,8 g (= 97 % d. Th.) 1-(3,6-dioxaheptylcarbamoyl)-4-methyl-2-benzimidazolyl-methylcarbamat der Molmasse 350 ($M^+$ im MS) als Feststoff mit einem Schmelzpunkt 187° C.

Beispiel A Vergleich der Hydrolysegeschwindigkeit

Die Hydrolyse-Abbaugeschwindigkeit wurde in i-Propanol/ Wasser 1 : 1 bei pH = 7 und 40°C bestimmt. Die Wirkstoffkonzentration bezieht sich auf den tatsächlichen Wirkstoffgehalt (± 2-3 ppm).

Tabelle

| Eingesetzte Verbindungen | Beispiel 1 (erfindungsgemäß) | 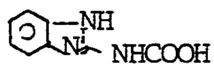 (bekannt) | Beispiel 4 (erfindungsgemäß) | 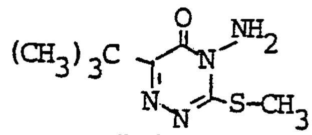 (bekannt) |
|---|---|---|---|---|
| Anfangskonzentr. | 100 ppm | 100 ppm | 100 ppm | 100 ppm |
| nach 15 d | 80 ppm | 70 ppm | 85 ppm | 75 ppm |
| 30 d | 70 ppm | 60 ppm | 75 ppm | 60 ppm |
| 45 d | 60 ppm | 50 ppm | 70 ppm | 55 ppm |
| 60 d | 55 ppm | 40 ppm | 65 ppm | 50 ppm |
| 90 d | 45 ppm | 20 ppm | 50 ppm | 25 ppm |

Patentansprüche

1. Verbindungen, dadurch gekennzeichnet, daß sie herstellbar sind indem man Schädlingsbekämpfungsmittel mit mindestens einem Zerewitinoff-aktiven Wasserstoffatom mit Polyetherisocyanaten umsetzt, unter der Voraussetzung, daß die Umsetzung mit dem Polyetherisocyanat nicht an einem Wasserstoffatom erfolgt, das Teil einer Carbaminsäureestergruppe ( $-O-\overset{O}{\overset{\|}{C}}-NH-$ ) ist.

2. Verfahren zur Herstellung von Verbindungen, dadurch gekennzeichnet, daß man Schädlingsbekämpfungsmittel mit mindestens einem Zerewitinoff-aktiven Wasserstoffatom mit Polyetherisocyanaten umsetzt, unter der Voraussetzung, daß die Umsetzung nicht an einem Wasserstoffatom erfolgt, das Teil einer Carbaminsäureestergruppe ( $-O-\overset{O}{\overset{\|}{C}}-NH-$ ) ist.

3. Verbindungen gemäß Anspruch 1, dadurch gekennzeichnet, daß als Schädlingsbekämpfungsmittel mit mindestens einem Zerewitinoff-aktiven Wasserstoffatom Verbindungen eingesetzt werden, die

   a) eine oder mehrere aminische Gruppen enthalten, die mindetens einen freien NH-Rest aufweisen, wobei der NH-Rest Teil eines Heterocyclus sein kann,

b) eine oder mehrere Hydrazingruppen enthalten, die mindestens einen freien NH-Rest aufweisen, wobei die Hydrazingruppe Teil eines Heterocyclus sein kann,

c) eine oder mehrere Guanidingruppen enthalten, die mindestens einen freien NH-Rest aufweisen, wobei die Guanidingruppe Teile eines Heterocyclus sein kann,

d) eine oder mehrere alkoholische Hydroxyl- oder Mercapto-Gruppen enthalten.

e) eine oder mehrere phenolische Hydroxyl- oder Mercapto-Gruppen enthalten.

f) eine oder mehrere Carbonsäureamid-Gruppen enthalten, die mindestens einen freien NH-Rest aufweisen wobei die Carbonsäureamidgruppe Teil eines Heterocyclus sein kann,

g) einen oder mehrere Harnstoff-Gruppen enthalten, die mindestens einen freien NH-Rest aufweisen, wobei die Harnstoffgruppe Teil eines Heterocyclus sein kann.

4. Verbindungen gemäß Anspruch 1, dadurch gekennzeichnet, daß als Schädlingsbekämpfungsmittel mit mindestens einem Zerewitinoff-aktiven Wasserstoffatom Verbindungen der allgemeinen Formel II eingesetzt werden

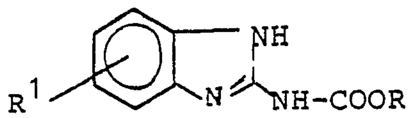

II

in welcher

R für $C_{1-4}$-Alkyl

$R^1$ für $C_{1-6}$-Alkyl oder Wasserstoff steht.

5. Verbindungen gemäß Anspruch 1, dadurch gekennzeichnet, daß als Polyetherisocyanate Verbindungen der Formel III

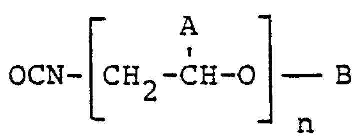

III

in welcher

A für Wasserstoff oder Methyl,

B für $C_{1-4}$-Alkyl oder den Rest $-C_{1-4}$-Alkylen-NCO,

n für ganze Zahlen zwischen 1 und 101 steht

eingesetzt werden.

6. Verbindung der Formel

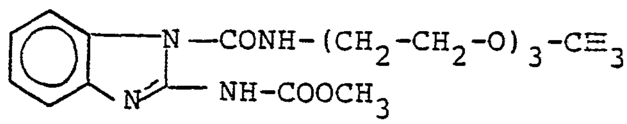

7. Schädlingsbekämpfungsmittel, gekennzeichnet durch einen Gehalt an mindestens einer Verbindung gemäß Anspruch 1.

8. Verwendung von Verbindungen gemäß Anspruch 1 zur Bekämpfung von Schädlingen.

9. Verfahren zur Bekämpfung von Schädlingen, dadurch gekennzeichnet, daß man Verbindungen gemäß Anspruch 1 auf Schädlinge und/oder ihren Lebensraum einwirken läßt.

10. Verfahren zur Herstellung von Schädlingsbekämpfungsmitteln, dadurch gekennzeichnet, daß man Verbindungen gemäß Anspruch 1 mit Streckmitteln und/oder oberflächenaktiven Mitteln vermischt.



BAD ORIGINAL